# EUROPEAN PATENT APPLICATION

(11) **EP 4 609 812 A1**
(43) Date of publication of application: **03.09.2025**
(21) Application number: 25157355.6
(22) Date of filing: 12.02.2025
(51) Int. Cl.: A61B 34/00, A61B 34/37, A61B 90/00, B25J 5/00, B25J 9/16, B25J 13/00, B25J 13/06, A61B 34/20

(54) **ROBOTIC SURGICAL SYSTEM**

(30) Priority: 01.03.2024 JP 2024030881
(71) Applicant: KAWASAKI JUKOGYO KABUSHIKI KAISHA, Kobe-shi, Hyogo 650-8670 (JP)
(72) Inventor: KOBAYASHI, Ayataka, Kobe-shi, Hyogo, 650-8670 (JP); MIZOHATA, Yuichi, Kobe-shi, Hyogo, 650-8670 (JP); YAMAMOTO, Daisuke, Kobe-shi, Hyogo, 650-8670 (JP)
(74) Representative: Hoffmann Eitle

(57) **Abstract**

A robotic surgical system (500) includes a controller (310) configured or programmed to restrict an operation to rotate a shaft (2d) in a direction out of a rotational operating range of the shaft (2d) with an operation unit (110) and an operation to bend a distal end device (2b) with respect to the shaft (2d) with the operation unit (110) when an operation for a surgical instrument (2) received by the operation unit (110) includes an operation to rotate the shaft (2d) out of the rotational operating range of the shaft (2d).

## Description

### BACKGROUND OF THE INVENTION

### Field of the Invention

The present disclosure relates to a robotic surgical system, a control method for a robotic surgical system, a program, and a storage medium.

### Description of the Background Art

Conventionally, a robotic surgical system including a surgical instrument attached to the distal end of a robot arm and an operation unit to receive an operation for the surgical instrument is known. Japanese Patent No. 6000641 discloses a master-slave manipulator including a slave manipulator arm to which a treatment tool is attached, a manipulation input device including a manipulation handle, and a controller. In Japanese Patent No. 6000641, the treatment tool is remotely controlled by the manipulation handle. In Japanese Patent No. 6000641, the treatment tool is a pair of forceps, for example. Furthermore, in Japanese Patent No. 6000641, the controller monitors a difference between a command value for opening and closing the pair of forceps from the manipulation handle and the actual opening and closing angle of the pair of forceps. When the difference between the command value for opening and closing the pair of forceps and the actual opening and closing angle of the pair of forceps exceeds an allowable value, an interlock mode in which a manipulation for the treatment tool with the manipulation handle is received transitions to an interlock stop mode in which a manipulation for the treatment tool with the manipulation handle is not received. Furthermore, in Japanese Patent No. 6000641, after the controller detects that the treatment tool has been opened to its maximum opening and closing angle by the manipulation handle in the interlock stop mode, the treatment tool is closed again with the manipulation handle, and the controller transitions again to the interlock mode based on the difference between the command value for opening and closing the pair of forceps and the actual opening and closing angle of the pair of forceps becoming equal to or smaller than the allowable value.

However, in Japanese Patent No. 6000641, the controller switches between the interlock stop mode and the interlock mode based on the difference between the command value for opening and closing the pair of forceps from the manipulation handle and the actual opening and closing angle of the pair of forceps, but Japanese Patent No. 6000641 does not mention a case in which a manipulation is performed by the manipulation handle that attempts to exceed the movable range of the treatment tool. When the interlock stop mode is set each time the manipulation handle exceeds the movable range of the treatment tool, surgery is interrupted each time and efficient surgery cannot be performed. On the other hand, when the interlock mode is maintained even when the manipulation handle exceeds the movable range of the treatment tool, the posture of the treatment tool does not change, but the posture of the manipulation handle changes. In such a case, when a manipulation received by the manipulation handle returns to a manipulation within the movable range of the treatment tool, the posture of the treatment tool changes suddenly to follow the posture of the manipulation handle. From the above, it is desired to reduce or prevent a sudden change in the posture of the treatment tool while the interlock mode is maintained, even when a manipulation is performed by the manipulation handle that exceeds the movable range of the treatment tool.

### SUMMARY OF THE INVENTION

The present disclosure is intended to provide a robotic surgical system, a control method for a robotic surgical system, a program, and a storage medium each capable of reducing or preventing a sudden change in the posture of a surgical instrument while a state in which an operation with an operation unit can be received is maintained even when an operation is performed that attempts to exceed the movable range of the surgical instrument.

A robotic surgical system according to a first aspect of the present disclosure includes a robot arm to allow a surgical instrument including a shaft and a distal end device connected to a distal end of the shaft via a wrist joint to be attached thereto, an operation apparatus including an operation unit to receive an operation for the surgical instrument, and a controller configured or programmed to restrict an operation to rotate the shaft in a direction out of a rotational operating range of the shaft with the operation unit and an operation to bend the distal end device with respect to the shaft with the operation unit when the operation for the surgical instrument received by the operation unit includes an operation to rotate the shaft out of the rotational operating range of the shaft.

In the robotic surgical system according to the first aspect of the present disclosure, the controller is configured or programmed to restrict the operation to rotate the shaft in the direction out of the rotational operating range of the shaft with the operation unit and the operation to bend the distal end device with respect to the shaft with the operation unit when the operation for the surgical instrument received by the operation unit includes the operation to rotate the shaft out of the rotational operating range of the shaft. Accordingly, even when an operation is performed to rotate the shaft out of the rotational operating range of the shaft, the operation to rotate the shaft in the direction out of the rotational operating range of the shaft with the operation unit and the operation to bend the distal end device with respect to the shaft with the operation unit are restricted, and thus the posture of the operation unit is maintained. Therefore, the possibility that the postures of the distal end device and the shaft do not match the posture of the operation unit is reduced or prevented, and thus a state in which the postures of the distal end device and the shaft match the posture of the operation unit is maintained. In addition, the controller only restricts the operation to rotate the shaft in the direction out of the rotational operating range with the operation unit and the operation to bend the distal end device with respect to the shaft with the operation unit, but does not restrict reception of all operations with the operation unit. Therefore, even when an operation is performed that attempts to exceed the movable range of the surgical instrument, a sudden change in the posture of the surgical instrument can be reduced or prevented while a state in which an operation with the operation unit can be received is maintained.

A control method for a robotic surgical system according to a second aspect of the present disclosure is a control method for a robotic surgical system including a robot arm to allow a surgical instrument including a shaft and a distal end device connected to a distal end of the shaft via a wrist joint to be attached thereto, and an operation apparatus including an operation unit to receive an operation for the surgical instrument, and includes receiving the operation for the surgical instrument by the operation unit, and restricting an operation to rotate the shaft in a direction out of a rotational operating range of the shaft with the operation unit and an operation to bend the distal end device with respect to the shaft with the operation unit when the operation for the surgical instrument received by the operation unit includes an operation to rotate the shaft out of the rotational operating range of the shaft.

As described above, the control method for the robotic surgical system according to the second aspect of the present disclosure includes restricting the operation to rotate the shaft in the direction out of the rotational operating range of the shaft with the operation unit and the operation to bend the distal end device with respect to the shaft with the operation unit when the operation for the surgical instrument received by the operation unit includes the operation to rotate the shaft out of the rotational operating range of the shaft. Accordingly, even when an operation is performed to rotate the shaft out of the rotational operating range of the shaft, the operation to rotate the shaft in the direction out of the rotational operating range of the shaft with the operation unit and the operation to bend the distal end device with respect to the shaft with the operation unit are restricted, and thus the posture of the operation unit is maintained. Therefore, the possibility that the postures of the distal end device and the shaft do not match the posture of the operation unit is reduced or prevented, and thus a state in which the postures of the distal end device and the shaft match the posture of the operation unit is maintained. In addition, the controller only restricts the operation to rotate the shaft in the direction out of the rotational operating range with the operation unit and the operation to bend the distal end device with respect to the shaft with the operation unit, but does not restrict reception of all operations with the operation unit. Therefore, it is possible to provide the control method for the robotic surgical system capable of reducing or preventing a sudden change in the posture of the surgical instrument while a state in which an operation with the operation unit can be received is maintained even when an operation is performed that attempts to exceed the movable range of the surgical instrument.

According to the present disclosure, even when an operation is performed that attempts to exceed the movable range of the surgical instrument, a sudden change in the posture of the surgical instrument can be reduced or prevented while a state in which an operation with the operation unit can be received is maintained.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

FIG. 1 is a diagram showing the configuration of a robotic surgical system according to an embodiment.
FIG. 2 is a diagram showing a display of a medical cart according to the embodiment.
FIG. 3 is a diagram showing the configuration of the medical cart according to the embodiment.
FIG. 4 is a diagram showing the configuration of a robot arm according to the embodiment.
FIG. 5 is a diagram showing an instrument.
FIG. 6 is a perspective view showing the configuration of an arm operation unit according to the embodiment.
FIG. 7 is a diagram for illustrating translational movement of the robot arm.
FIG. 8 is a diagram for illustrating rotational movement of the robot arm.
FIG. 9 is a diagram showing an endoscope.
FIG. 10 is a diagram showing a pivot position setting instrument.
FIG. 11 is a diagram showing operation units according to the embodiment.
FIG. 12 is a diagram showing a right-handed wrist according to the embodiment.
FIG. 13 is a diagram showing a left-handed wrist according to the embodiment.
FIG. 14 is a perspective view showing foot pedals according to the embodiment.
FIG. 15 is a control block diagram of the robotic surgical system according to the embodiment.
FIG. 16 is a control block diagram of the robot arm according to the embodiment.
FIG. 17 is a control block diagram of a positioner and the medial cart according to the embodiment.
FIG. 18 is a control block diagram of the operation unit according to the embodiment.
FIG. 19 is a control flow diagram of a first controller for an operation to rotate a shaft out of its rotational operating range.
FIG. 20 is a diagram showing a state in which indicators indicating the movable ranges of the robot arms, the movable ranges of the operation units, and the current positions of the robot arms are displayed on a monitor and a display.
FIG. 21 is a diagram showing the indicator indicating the movable range of the robot arm, the movable range of the operation unit, and the current position of the robot arm.
FIG. 22 is a diagram showing a state in which a message is displayed, indicating that the operation is outside the rotational operating range of the shaft.

### DESCRIPTION OF THE PREFERRED EMBODIMENTS

### Configuration of Robotic Surgical System

The configuration of a robotic surgical system 500 according to this embodiment is now described. The robotic surgical system 500 includes a surgical robot 100, a remote control apparatus 200, a vision unit 300, and an image processing unit 400. The remote control apparatus 200 is an example of an operation apparatus.

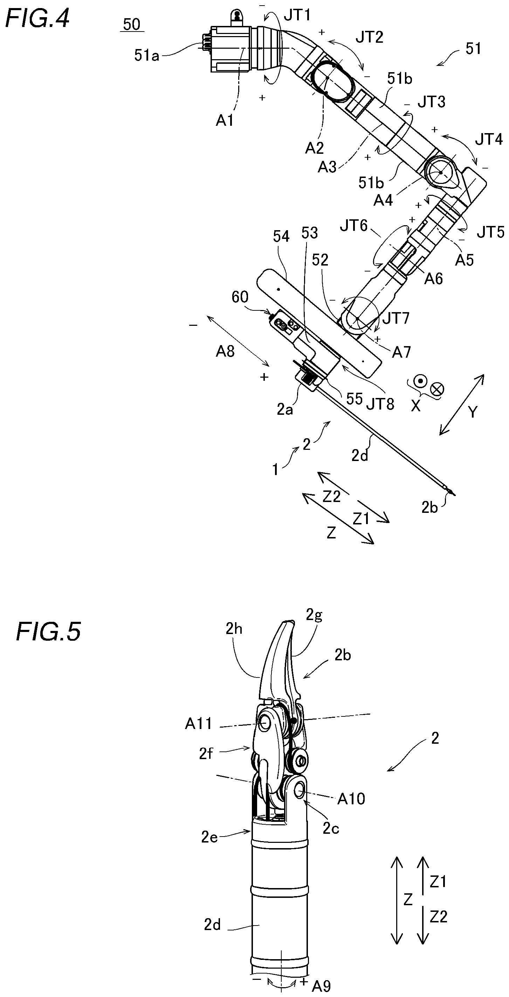

In this specification, as shown in FIG. 4, the longitudinal direction of a surgical instrument 1 is defined as a Z direction. The distal end side of the surgical instrument 1 is defined as a Z1 side, and the proximal end side of the surgical instrument 1 is defined as a Z2 side. A direction perpendicular to the Z direction is defined as an X direction. A direction perpendicular to the Z direction and the X direction is defined as a Y direction.

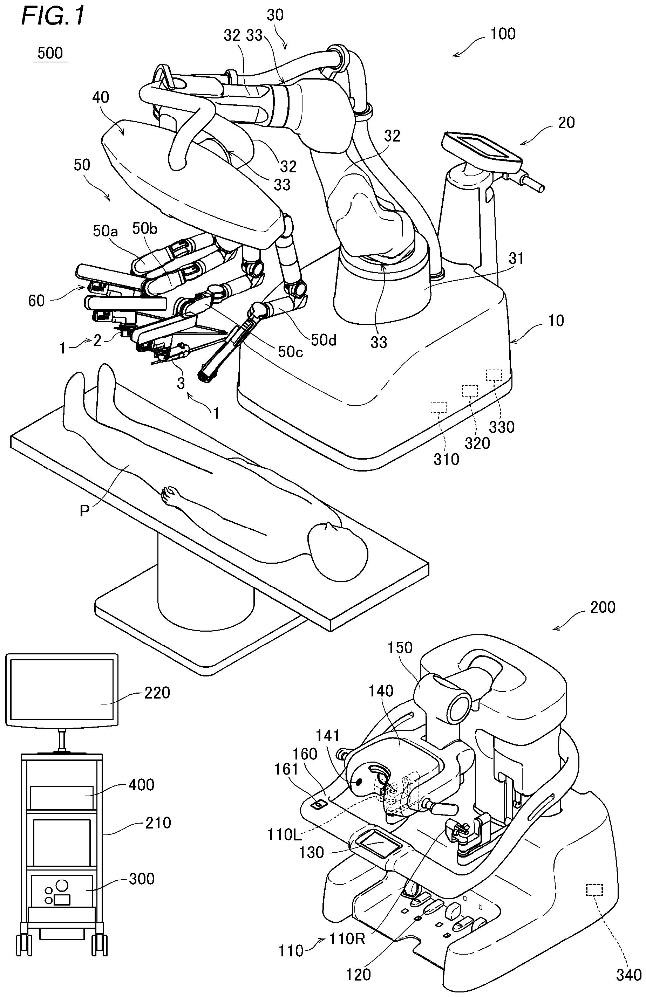

As shown in FIG. 1, the surgical robot 100 is arranged in an operating room. The remote control apparatus 200 is spaced apart from the surgical robot 100. The remote control apparatus 200 receives operations for the surgical instrument 1. Specifically, an operator such as a doctor inputs a command to the remote control apparatus 200 to cause the surgical robot 100 to perform a desired operation. The remote control apparatus 200 transmits the input command to the surgical robot 100. The surgical robot 100 operates based on the received command. The surgical robot 100 is arranged in the operating room that is a sterilized sterile field.

### Configuration of Surgical Robot

As shown in FIG. 1, the surgical robot 100 includes a medical cart 10, a cart positioner operation unit 20, a positioner 30, an arm base 40, a plurality of robot arms 50, and an arm operation unit 60 provided on each of the robot arms 50.

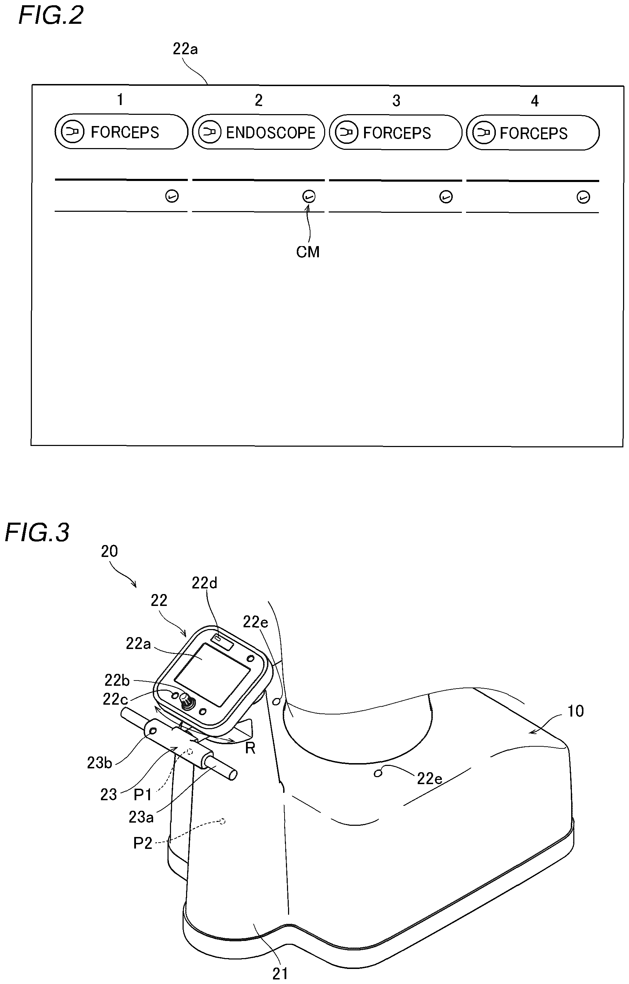

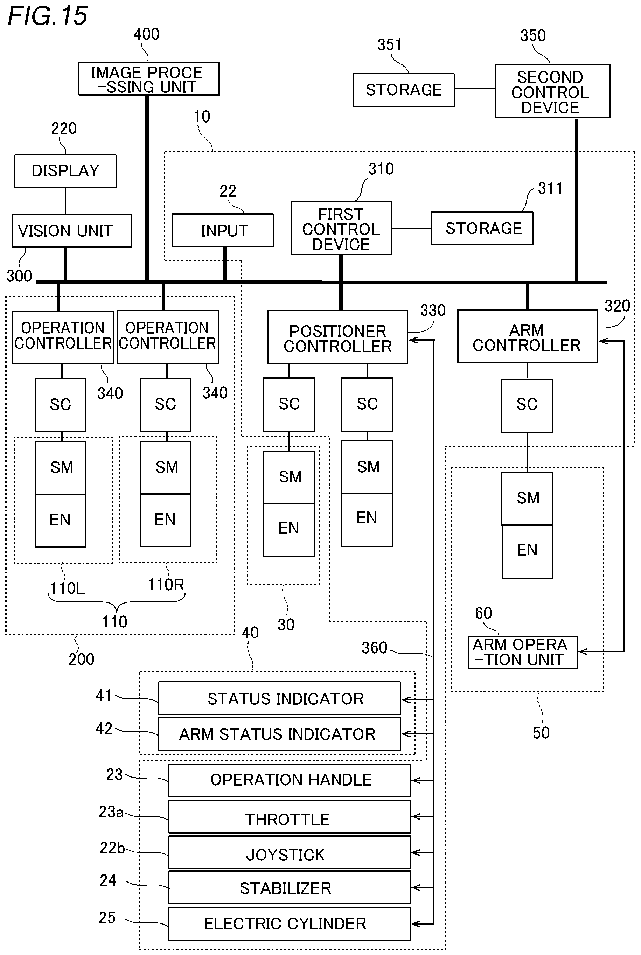

As shown in FIG. 3, the cart positioner operation unit 20 is supported by a cart positioner operation support 21 at the rear of the medical cart 10, and the medical cart 10 or the positioner 30 is moved by operating the cart positioner operation unit 20. The cart positioner operation unit 20 includes an input 22 and an operation handle 23. The input 22 receives operations to move the positioner 30, the arm base 40, and the plurality of robot arms 50 or change their postures mainly in order to prepare for surgery before the surgery. The medical cart 10 includes the operation handle 23, and a stabilizer 24 and an electric cylinder 25 shown in FIG. 15.

As shown in FIG. 3, the input 22 of the medical cart 10 includes a display 22a, a joystick 22b, an enable switch 22c, an error reset button 22d, and speakers 22e. The display 22a is a liquid crystal panel, for example. As shown in FIG. 2, the display 22a displays numbers corresponding to the plurality of robot arms 50. The display 22a also displays the type of surgical instrument 1 attached to each of the plurality of robot arms 50. A check mark CM indicating that a pivot position PP described below has been set is displayed on the display 22a.

As shown in FIG. 3, the joystick 22b is arranged in the vicinity of or adjacent to the display 22a of the input 22 of the medical cart 10. The positioner 30 is moved three-dimensionally by selecting an operation mode displayed on the display 22a and operating the joystick 22b.

The enable switch 22c is arranged in the vicinity of or adjacent to the joystick 22b. The enable switch 22c enables or disables movement of the positioner 30. When the joystick 22b is operated while the enable switch 22c is pressed to enable movement of the positioner 30, the positioner 30 is moved.

The error reset button 22d resets errors in the robotic surgical system 500. The errors may be abnormal deviation errors, for example. The speakers 22e are arranged in pair. The pair of speakers 22e are arranged in the vicinity of or adjacent to the location of the positioner 30 in the medical cart 10.

The operation handle 23 is arranged in the vicinity of the display 22a. The operation handle 23 includes a throttle 23a that is gripped and twisted by an operator such as a nurse or a technician to operate movement of the medical cart 10. Specifically, the operation handle 23 is arranged below the input 22. As the throttle 23a is twisted from the near side to the far side, the medical cart 10 moves forward. As the throttle 23a is twisted from the far side to the near side, the medical cart 10 moves rearward. The speed of the medical cart 10 is changed according to a twisting amount of the throttle 23a. The operation handle 23 is rotatable to the left and right shown by an R direction, and the medical cart 10 is turned with rotation of the operation handle 23.

An enable switch 23b for enabling or disabling movement of the medical cart 10 is provided on the operation handle 23 of the cart positioner operation unit 20. When the throttle 23a of the operation handle 23 is operated while the enable switch 23b is pressed to enable movement of the medical cart 10, the medical cart 10 is moved.

As shown in FIG. 1, the positioner 30 includes a 7-axis articulated robot, for example. The positioner 30 is arranged on the medical cart 10. The positioner 30 adjusts the position of the arm base 40. The positioner 30 moves the position of the arm base 40 three-dimensionally.

The positioner 30 includes a base 31 and a plurality of links 32 coupled to the base 31. The plurality of links 32 are coupled to each other by joints 33.

The arm base 40 is attached to the distal end of the positioner 30. The proximal end of each of the plurality of robot arms 50 is attached to the arm base 40. Each of the plurality of robot arms 50 is able to take a folded and stored posture. The arm base 40 and the plurality of robot arms 50 are covered with sterile drapes and used. Moreover, each of the robot arms 50 supports the surgical instrument 1.

A status indicator 41 and an arm status indicator 42 that are shown in FIG. 15 are provided on the arm base 40. The status indicator 41 indicates the status of the robotic surgical system 500. The arm status indicator 42 indicates the statuses of the robot arms 50.

The plurality of robot arms 50 are arranged. Specifically, four robot arms 50a, 50b, 50c, and 50d are arranged. The robot arms 50a, 50b, 50c, and 50d have the same or similar configurations as each other.

As shown in FIG. 4, each robot arm 50 includes an arm portion 51, a first link 52, a second link 53, and a translation mechanism 54. The robot arm 50 includes joints JT1, JT2, JT3, JT4, JT5, JT6, JT7, and JT8. The joints JT1, JT2, JT3, JT4, JT5, JT6, and JT7 have A1, A2, A3, A4, A5, A6, and A7 axes as rotation axes, respectively. The joint JT8 has an A8 axis as a linear motion axis. The arm portion 51 includes a base 51a and links 51b.

The arm portion 51 includes a 7-axis articulated robot arm. The first link 52 is arranged at the distal end of the arm portion 51. The arm operation unit 60 described below is attached to the second link 53. The translation mechanism 54 is arranged between the first link 52 and the second link 53. A holder 55 that holds the surgical instrument 1 is arranged on the second link 53. The translation mechanism 54 translates the holder 55 to which the surgical instrument 1 is attached between a first position and a second position. The first position refers to an end position on the Z2 side in a range of movement of the holder 55 along the A8 axis by the translation mechanism 54. The second position refers to an end position on the Z1 side in the range of movement of the holder 55 along the A8 axis by the translation mechanism 54.

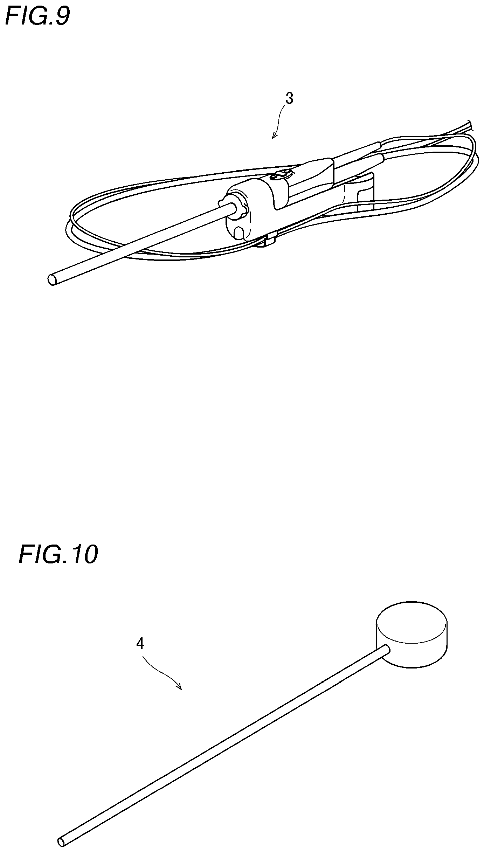

The surgical instrument 1 is attached to the distal end of each of the plurality of robot arms 50. The surgical instrument 1 includes a replaceable instrument 2, an endoscope 3 shown in FIG. 9 to capture an image of a surgical site, and a pivot position setting instrument 4 shown in FIG. 10 to set the pivot position PP, for example. The instrument 2 includes a driven unit 2a, an end effector 2b, and a wrist joint 2c and a shaft 2d shown in FIG. 5. The end effector 2b is connected to the distal end of the shaft 2d via the wrist joint 2c. The instrument 2 and the end effector 2b are examples of a surgical instrument and a distal end device, respectively.

As shown in FIG. 1, the endoscope 3 is attached to the distal end of one of the plurality of robot arms 50, such as the robot arm 50c, and the instrument 2 is attached to the distal end of each of the remaining robot arms 50a, 50b, and 50d, for example. The endoscope 3 is preferably attached to one of two robot arms 50b and 50c arranged in the center among the four robot arms 50 arranged adjacent to each other.

### Configuration of Instrument

As shown in FIG. 5, the end effector 2b including jaw members 2g and 2h is attached to the distal end of the instrument 2, for example. As the end effector 2b, a pair of scissors, a grasper, a needle holder, a microdissector, a stable applier, a tacker, a suction cleaning tool, a snare wire, a clip applier, etc. can be used.

The instrument 2 includes a first support member 2e and a second support member 2f. The first support member 2e is attached to the shaft 2d. The second support member 2f is supported by the first support member 2e so as to be rotatable around an A10 axis, and supports the end effector 2b such that the end effector 2b is rotatable around an A11 axis that intersects with the A10 axis as viewed in a direction in which the shaft 2d extends. The shaft 2d rotates around an A9 axis. The wrist joint 2c is provided between the second support member 2f and the first support member 2e with the A10 axis as a rotation axis. The A10 axis and the A11 axis are examples of a first rotation axis and a second rotation axis, respectively.

### Configuration of Arm Operation Unit

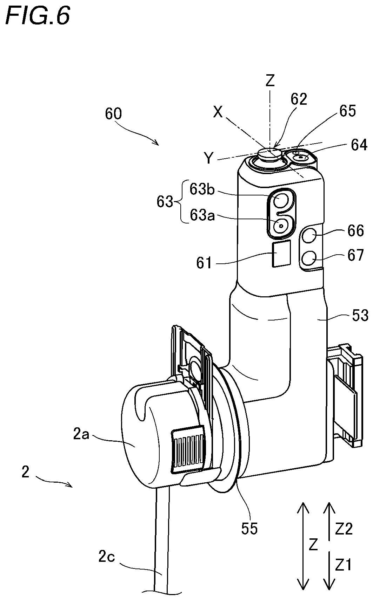

As shown in FIG. 6, the arm operation unit 60 is attached to the robot arm 50 to operate the robot arm 50. Specifically, the arm operation unit 60 is attached to the second link 53.

The arm operation unit 60 includes an enable switch 61, a joystick 62, and linear switches 63, a mode switching button 64, a mode indicator 65, a pivot button 66, and an adjustment button 67.

The enable switch 61 is pressed to enable or disable movement of the robot arm 50 in response to the joystick 62 and the linear switches 63. When the enable switch 61 is pressed by an operator such as a nurse or an assistant grasping the arm operation unit 60, movement of the surgical instrument 1 by the robot arm 50 is enabled.

The joystick 62 is an operation tool to control movement of the surgical instrument 1 by the robot arm 50. The joystick 62 controls a moving direction and a moving speed of the robot arm 50. The robot arm 50 is moved in accordance with a tilting direction and a tilting angle of the joystick 62.

The linear switches 63 are switches to move the surgical instrument 1 in the Z direction, which is the longitudinal direction of the surgical instrument 1. The linear switches 63 include a linear switch 63a to move the surgical instrument 1 in a direction in which the surgical instrument 1 is inserted into a patient P, and a linear switch 63b to move the surgical instrument 1 in a direction in which the surgical instrument 1 is moved away from the patient P. Both the linear switch 63a and the linear switch 63b are push-button switches.

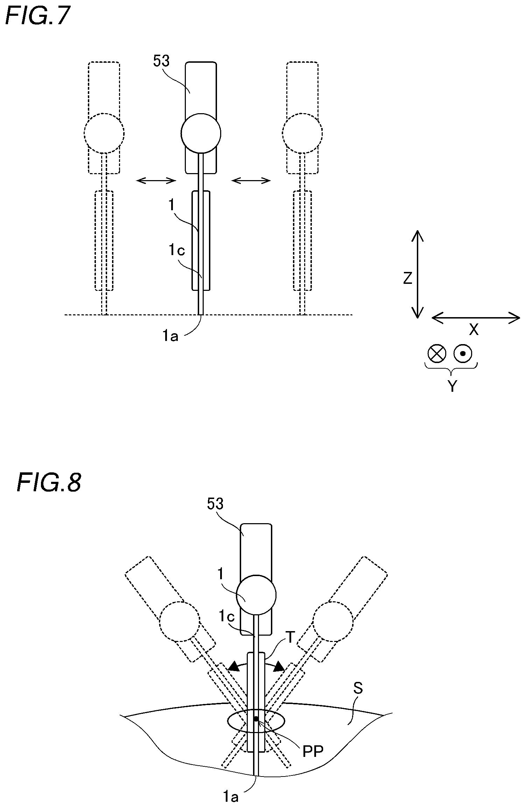

The mode switching button 64 is a push-button switch to switch between a mode for translationally moving the surgical instrument 1 and a mode for rotationally moving the surgical instrument 1. As shown in FIG. 7, in the mode for translationally moving the robot arm 50, the robot arm 50 is moved such that the distal end 1a of the surgical instrument 1 is moved in an X-Y plane. As shown in FIG. 8, in the mode for rotationally moving the robot arm 50, the robot arm 50 is moved such that the surgical instrument 1 is rotationally moved around a center on the A11 axis of the end effector 2b or the distal end of the end effector 2b of the instrument 2 as the surgical instrument 1 as a fulcrum when any pivot position PP is not stored in a storage 351, and the surgical instrument 1 is rotationally moved around the pivot position PP as a fulcrum when the pivot position PP is stored in the storage 351. In this case, the surgical instrument 1 is rotationally moved with the shaft 1c of the surgical instrument 1 inserted into a trocar T. The mode switching button 64 is arranged on a Z-direction side surface of the arm operation unit 60.

The mode indicator 65 indicates a switched mode. The mode indicator 65 is on to indicate a rotational movement mode and is off to indicate a translational movement mode. Furthermore, the mode indicator 65 also serves as a pivot position indicator that indicates that the pivot position PP has been set. The mode indicator 65 is arranged on the Z-direction side surface of the arm operation unit 60.

The pivot button 66 is a push-button switch to set the pivot position PP that serves as a fulcrum for movement of the surgical instrument 1 attached to the robot arm 50.

The adjustment button 67 is a button to optimize the position of the robot arm 50. After the pivot position PP for the robot arm 50 to which the endoscope 3 has been attached is set, the positions of the other robot arms 50 and the arm base 40 are optimized when the adjustment button 67 is pressed. The adjustment button 67 is a button different from the enable switch 61.

### Remote Control Apparatus

As shown in FIG. 1, the remote control apparatus 200 is arranged inside or outside the operating room, for example. The remote control apparatus 200 includes the operation units 110, foot pedals 120, a touch panel 130, a monitor 140, a support arm 150, a support bar 160, an error reset button 161. The operation units 110 include operation handles for the operator such as a doctor to input a command. The monitor 140 is an example of a first display or a second display.

### Operation Unit

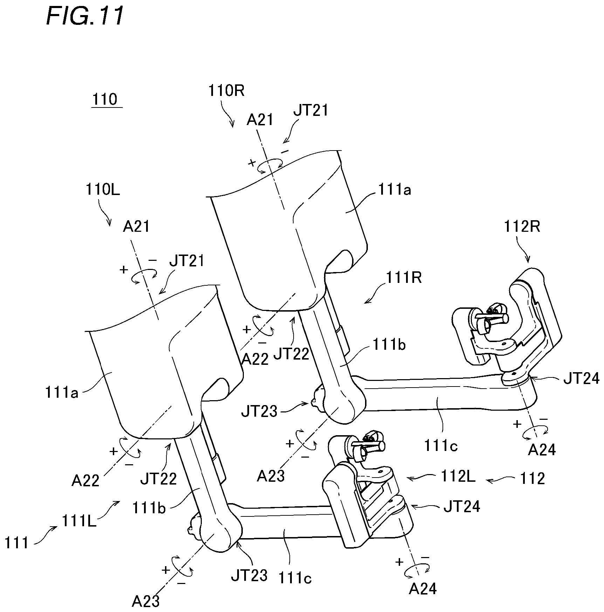

As shown in FIG. 11, the operation units 110 each include a handle to operate the surgical instrument 1. The operation unit 110 receives an operation for the surgical instrument 1. The operation units 110 include an operation unit 110L that is located on the left side as viewed from the operator such as a doctor and is to be operated by the left hand of the operator, and an operation unit 110R that is located on the right side and is to be operated by the right hand of the operator. The operation units 110 include arms 111 and wrists 112. The operation unit 110R includes an arm 111R and a wrist 112R. The operation unit 110L includes an arm 111L and a wrist 112L.

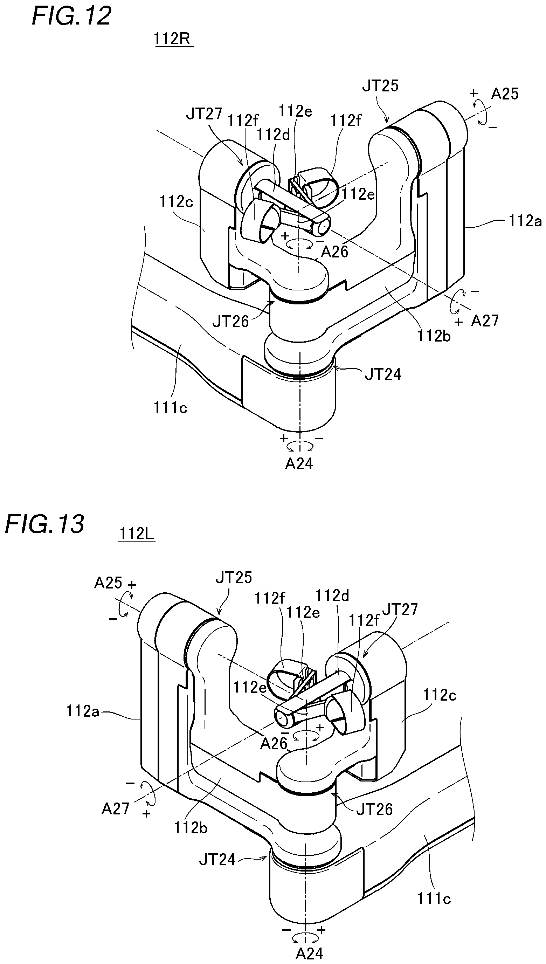

The operation units 110 each have joints JT21, JT22, and JT23 shown in FIG. 11, and joints JT24, JT25, JT26, and JT27 shown in FIGS. 12 and 13. The rotation axes of the joints JT21, JT22, JT23, JT24, JT25, JT26, and JT27 are A21, A22, A23, A24, A25, A26, and A27 axes, respectively.

As shown in FIG. 11, the arm 111R includes a link 111a, a link 111b, and a link 111c. The upper end side of the link 111a is attached to the remote control apparatus 200 such that the link 111a is rotatable around the A21 axis along a vertical direction. The upper end side of the link 111b is attached to the lower end side of the link 111a such that the link 111b is rotatable around the A22 axis along a horizontal direction. A first end side of the link 111c is attached to the lower end side of the link 111b such that the link 111c is rotatable around the A23 axis along the horizontal direction. The wrist 112 is attached to a second end side of the link 111c such that the wrist 112 is rotatable around the A24 axis. The link 111a is connected to the remote control apparatus 200 by the joint JT21. The link 111a and the link 111b are connected to each other by the joint JT22. The link 111b and the link 111c are connected to each other by the joint JT23. The arm 111 supports the wrist 112. The arm 111L has the same or similar configuration as the arm 111R.

The wrists 112 include the wrist 112R shown in FIG. 12 and to be operated by the right hand of the operator, and the wrist 112L shown in FIG. 13 and to be operated by the left hand of the operator. FIG. 12 shows the reference posture of the operation unit 110R, and FIG. 13 shows the reference posture of the operation unit 110L. The configuration of the wrist 112R is the same as or similar to that of the wrist 112L.

The wrists 112 each include a link 112a, a link 112b, a link 112c, and a grip support member 112d that is to be operated by the operator such as a doctor. The link 112a includes a proximal end connected to the distal end of the arm 111 and rotates around the A24 axis. The link 112b includes a proximal end connected to the distal end of the link 112a and rotates around the A25 axis with respect to the link 112a. The link 112c includes a proximal end connected to the distal end of the link 112b and a distal end to which the grip support member 112d is connected, and rotates around the A26 axis with respect to the link 112b. The grip support member 112d rotates around the A27 axis with respect to the link 112c. The link 112a, the link 112b, and the link 112c each have an L shape. The link 112a, the link 112b, and the link 112c are examples of a first link, a second link, and a third link, respectively.

The wrist 112 includes a pair of grip members 112e that are opened and closed by the operator. The grip members 112e each include an elongated plate-shaped lever member, and the proximal ends of the pair of grip members 112e are rotatably connected to the proximal end of the grip support member 112d. Cylindrical finger insertion portions 112f are provided on the grip members 112e. The operator inserts their fingers into a pair of finger insertion portions 112f to operate the wrist 112. The proximal ends of the pair of grip members 112e are connected to the grip support member 112d, and an angle between the pair of grip members 112e is increased or decreased such that an opening angle between the jaw member 2g and the jaw member 2h is changed. A magnet is provided on one of the grip members 112e, and a Hall sensor is provided on the grip support member 112d. When the operator opens and closes the grip members 112e, the magnet and the Hall sensor function as an angle detection sensor, and the Hall sensor outputs the opening angle. As the angle detection sensor, the Hall sensor may be provided on the grip member 112e, and the magnet may be provided on the grip support member 112d. Alternatively, the magnet or the Hall sensor may be provided as the angle detection sensor on both the grip members 112e.

As shown in FIG. 1, the monitor 140 is a scope-type display that displays images captured by the endoscope 3. A notifier 141 is provided on the monitor 140. The notifier 141 issues an error sound. The support arm 150 supports the monitor 140 so as to align the height of the monitor 140 with the height of the face of the operator such as a doctor. The touch panel 130 is arranged on the support bar 160. The head of the operator is detected by a sensor provided in the vicinity of the monitor 140 such that the surgical robot 100 can be operated by the remote control apparatus 200. The operator operates the operation units 110 and the foot pedals 120 while visually recognizing an affected area on the monitor 140. Thus, a command is input to the remote control apparatus 200. The command input to the remote control apparatus 200 is transmitted to the surgical robot 100.

### Foot Pedals

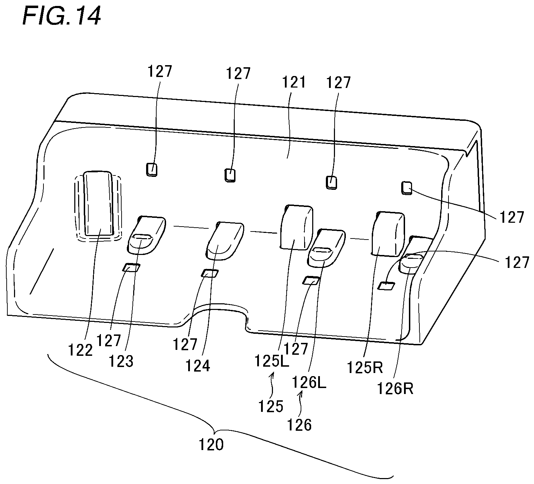

As shown in FIG. 14, a plurality of foot pedals 120 are provided to perform functions related to the surgical instrument 1. The plurality of foot pedals 120 are arranged on a base 121. The foot pedals 120 include a switching pedal 122, a clutch pedal 123, a camera pedal 124, incision pedals 125, coagulation pedals 126, and foot detectors 127. The switching pedal 122, the clutch pedal 123, the camera pedal 124, the incision pedals 125, and the coagulation pedals 126 are operated by the foot of the operator. The incision pedals 125 include an incision pedal 125R for a right robot arm 50, and an incision pedal 125L for a left robot arm 50. The coagulation pedals 126 include a coagulation pedal 126R for the right robot arm 50 and a coagulation pedal 126L for the left robot arm 50.

The switching pedal 122 switches robot arms 50 to be operated by the operation units 110. The clutch pedal 123 performs a clutch operation to temporarily disconnect an operation connection between the robot arms 50 and the operation units 110. While the clutch pedal 123 is being pressed by the operator, operations by the operation units 110 are not transmitted to the robot arms 50. While the camera pedal 124 is being pressed by the operator, the operation unit 110 can operate a robot arm 50 to which the endoscope 3 is attached. While the incision pedals 125 or the coagulation pedals 126 are being pressed by the operator, an electrosurgical device is activated.

### Vision Unit and Image Processing Unit

As shown in FIG. 1, the vision unit 300 and the image processing unit 400 are placed on a cart 210. The image processing unit 400 processes images captured by the endoscope 3. A display 220 is arranged on the cart 210. The display 220 displays images captured by the endoscope 3. The display 220 is an example of a first display or a second display.

### Configuration of Control System

As shown in FIG. 15, the robotic surgical system 500 includes a first control device 310, an arm controller 320, a positioner controller 330, operation controllers 340, and a second control device 350. The robotic surgical system 500 also includes a storage 311 connected to the first control device 310 and the storage 351 connected to the second control device 350. The first control device 310 is an example of a controller.

The first control device 310 is accommodated in the medical cart 10 to communicate with the arm controller 320 and the positioner controller 330, and controls the entire robotic surgical system 500. Specifically, the first control device 310 communicates with and controls the arm controller 320, the positioner controller 330, and the operation controllers 340. The first control device 310 is connected to the arm controller 320, the positioner controller 330, and the operation controllers 340 through a LAN, for example. The first control device 310 is arranged inside the medical cart 10.

The arm controller 320 is arranged for each of the plurality of robot arms 50. That is, the same number of arm controllers 320 as the plurality of robot arms 50 are placed inside the medical cart 10.

As shown in FIG. 15, the input 22 is connected to the first control device 310 through a LAN, for example. The status indicator 41, the arm status indicator 42, the operation handle 23, the throttle 23a, the joystick 22b, the stabilizer 24, and the electric cylinder 25 are connected to the positioner controller 330 via a wire line 360 by means of a communication network that allows information to be shared with each other by using serial communication. Although FIG. 15 shows that the status indicator 41, the arm status indicator 42, etc. are all connected to one wire line 360, in reality, the wire line 360 is arranged for each of the status indicator 41, the arm status indicator 42, the operation handle 23, the throttle 23a, the joystick 22b, the stabilizer 24, and the electric cylinder 25.

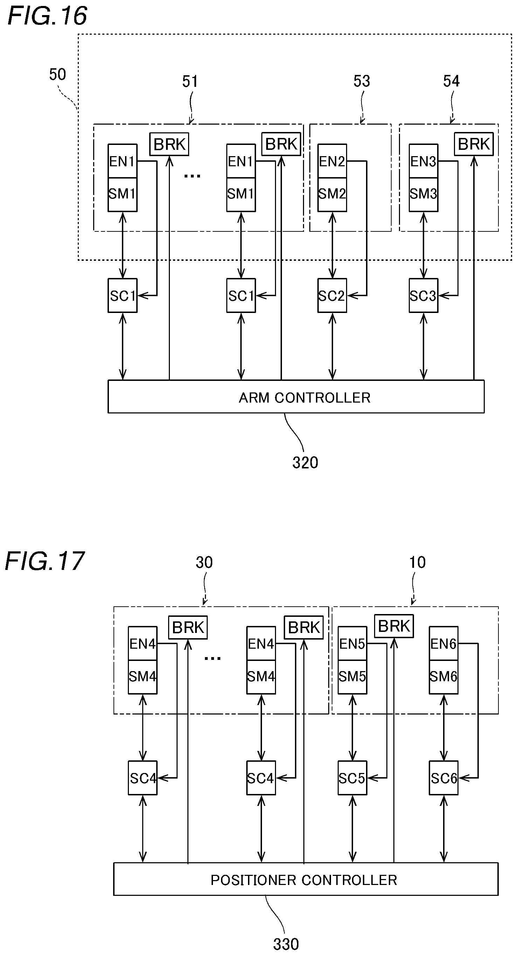

As shown in FIG. 16, the arm portion 51 includes a plurality of servomotors SM1, encoders EN1, and speed reducers so as to correspond to the joints JT1, JT2, JT3, JT4, JT5, JT6, and JT7. The encoders EN1 detect rotation angles of the servomotors SM1. The speed reducers slow down rotation of the servomotors SM1 to increase the torques. Inside the medical cart 10, servo controllers SC1 that control the servomotors SM1 are arranged adjacent to the arm controller 320. In addition, the encoders EN1 that detect the rotation angles of the servomotors SM1 are electrically connected to the servo controllers SC1.

Servomotors SM2 that rotate driven members provided in the driven unit 2a of the surgical instrument 1, encoders EN2, and speed reducers are arranged in the second link 53. The encoders EN2 detect rotation angles of the servomotors SM2. The speed reducers slow down rotation of the servomotors SM2 to increase the torques. In the medical cart 10, servo controllers SC2 are provided to control the servomotors SM2 to drive the surgical instrument 1. The encoders EN2 that detect the rotation angles of the servomotors SM2 are electrically connected to the servo controllers SC2. A plurality of servomotors SM2, a plurality of encoders EN2, and a plurality of servo controllers SC2 are arranged.

The translation mechanism 54 includes a servomotor SM3 to translationally move the surgical instrument 1, an encoder EN3, and a speed reducer. The encoder EN3 detects a rotation angle of the servomotor SM3. The speed reducer slows down rotation of the servomotor SM3 to increase the torque. In the medical cart 10, a servo controller SC3 is provided to control the servomotor SM3 to translationally move the surgical instrument 1. The encoder EN3 that detects the rotation angle of the servomotor SM3 is electrically connected to the servo controller SC3.

The first control device 310 generates command values to command the positions of the servomotors SM1, SM2, and SM3 based on operations received by the remote control apparatus 200, and drives the servomotors SM1, SM2, and SM3 based on the command values. The first control device 310 detects an abnormal deviation error when differences between the command values and the positions of the servomotors SM1, SM2, and SM3 detected by sensors exceed an allowable range.

As shown in FIG. 17, a plurality of servomotors SM4, a plurality of encoders EN4, and a plurality of speed reducers are provided in the positioner 30 so as to correspond to the plurality of joints 33 of the positioner 30. The encoders EN4 detect rotation angles of the servomotors SM4. The speed reducers slow down rotation of the servomotors SM4 to increase the torques.

The medical cart 10 includes wheels including front wheels as drive wheels and rear wheels that are steered by the operation handle 23. The rear wheels are arranged closer to the operation handle 23 than the front wheels. The medical cart 10 includes servomotors SM5 to drive a plurality of front wheels of the medical cart 10, encoders EN5, speed reducers, and brakes BRK. The speed reducers slow down rotation of the servomotors SM5 to increase the torques. A potentiometer P1 shown in FIG. 3 is provided on the operation handle 23 of the medical cart 10, and the servomotors SM5 of the front wheels are driven based on a rotation angle detected by the potentiometer P1 according to the twist of the throttle 23a. Rear wheels of the medical cart 10 are of the dual wheel type, and the rear wheels are steered based on rightward-leftward rotation of the operation handle 23. Furthermore, a potentiometer P2 shown in FIG. 3 is provided on a rotation axis of the operation handle 23 of the medical cart 10, and servomotors SM6, encoders EN6, and speed reducers are provided on the rear wheels of the medical cart 10. The speed reducers slow down rotation of the servomotors SM6 to increase the torques. The servomotors SM6 are driven based on a rotation angle detected by the potentiometer P2 according to rightward-leftward rotation of the operation handle 23. That is, steering of the rear wheels by the rightward-leftward rotation of the operation handle 23 is power-assisted by the servomotors SM6.

The front wheels of the medical cart 10 are driven such that the medical cart 10 moves in a forward-rearward direction. Furthermore, the operation handle 23 of the medical cart 10 is rotated such that the rear wheels are steered, and the medical cart 10 turns in a right-left direction.

As shown in FIG. 17, in the medical cart 10, servo controllers SC4 are provided to control the servomotors SM4 to move the positioner 30. The encoders EN4 that detect the rotation angles of the servomotors SM4 are electrically connected to the servo controllers SC4. In the medical cart 10, servo controllers SC5 are provided to control the servomotors SM5 to drive the front wheels of the medical cart 10. The encoders EN5 that detect the rotation angles of the servomotors SM5 are electrically connected to the servo controllers SC5. In the medical cart 10, servo controllers SC6 are provided to control the servomotors SM6 to power-assist steering of the rear wheels of the medical cart 10. The encoders EN6 that detect the rotation angles of the servomotors SM6 are electrically connected to the servo controllers SC6.

As shown in FIGS. 16 and 17, the joints JT1, JT2, JT3, JT4, JT5, JT6, and JT7 of the arm portion 51, and the joints 33 of the positioner 30 include brakes BRK. Furthermore, the front wheels of the medical cart 10, the arm base 40, and the translation mechanism 54 include brakes BRK. The arm controller 320 unidirectionally transmits control signals to the brakes BRK of the joints JT1, JT2, JT3, JT4, JT5, JT6, and JT7 of the arm portion 51, and the translation mechanism 54. The control signals are signals for turning on/off the brakes BRK. The signals for turning on the brakes BRK include signals for maintaining the brakes BRK in an enabled state. The same applies to control signals from the positioner controller 330 to the brakes BRK of the joints 33 of the positioner 30 and the arm base 40. On startup, all the brakes BRK of the arm base 40, the arm portion 51, and the translation mechanism 54 are turned off but the servomotors SM are driven against gravity to maintain the postures of the robot arm 50 and the arm base 40. When an error occurs in the robotic surgical system 500, the brakes BRK of the arm base 40, the arm portion 51 and the translation mechanism 54 are turned on. When the error in the robotic surgical system 500 is reset, the brakes BRK of the arm base 40, the arm portion 51, and the translation mechanism 54 are turned off. When a shutdown operation is performed in the robotic surgical system 500, the brakes BRK of the arm base 40, the arm portion 51, and the translation mechanism 54 are turned on. The brakes BRK of the front wheels of the medical cart 10 are constantly turned on, and the brakes BRK are deactivated only while the enable switch 23b of the medical cart 10 is being pressed. The brakes BRK of the joints 33 of the positioner 30 are constantly turned on, and the brakes BRK are deactivated only while the enable switch 22c of the medical cart 10 is being pressed.

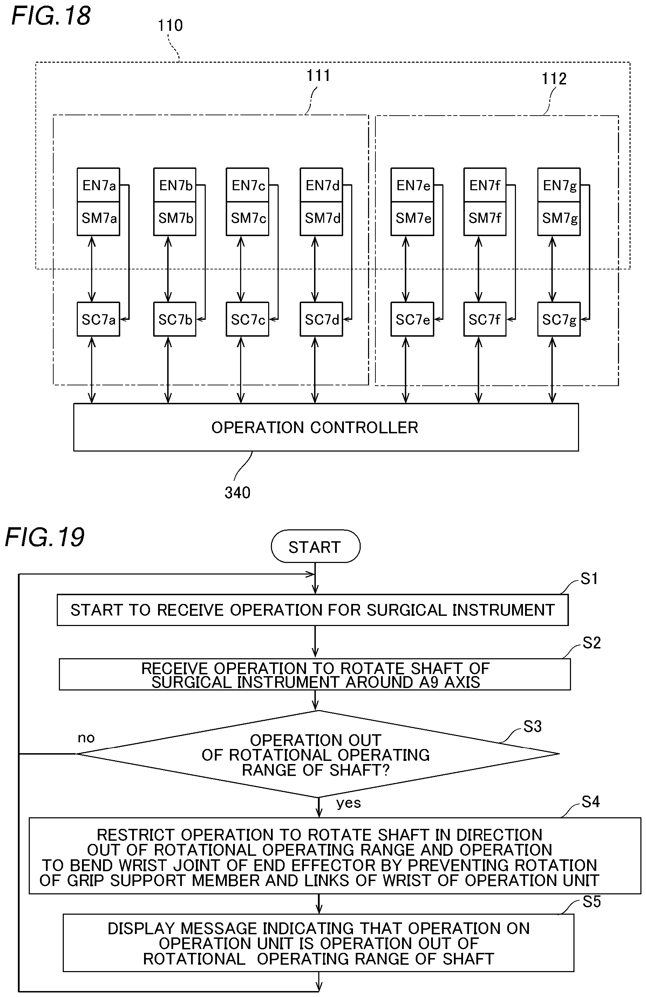

As shown in FIG. 18, servomotors SM7a, SM7b, SM7c, SM7d, SM7e, SM7f, and SM7g are arranged on the joints JT21, JT22, JT23, JT24, JT25, JT26, and JT27 of the operation unit 110, respectively. The servomotor SM7a rotates the link 111a around the A21 axis. The servomotor SM7b rotates the link 111b around the A22 axis. The servomotor SM7c rotates the link 111c around the A23 axis. The servomotor SM7d rotates the link 112a around the A24 axis. The servomotor SM7e rotates the link 112b around the A25 axis. The servomotor SM7f rotates the link 112c around the A26 axis. The servomotor SM7g rotates the grip support member 112d around the A27 axis. The A24 axis, the A25 axis, and the A26 axis are examples of a fourth rotation axis, a fifth rotation axis, and a sixth rotation axis, respectively. The A27 axis is an example of a third rotation axis. Servo controllers SC7a, SC7b, SC7c, SC7d, SC7e, SC7f, and SC7g are provided to control the servomotors. Encoders EN7a, EN7b, EN7c, EN7d, EN7e, EN7f, and EN7g are electrically connected to the servo controllers to detect rotation angles of the servomotors. The servomotors, the servo controllers, and the encoders are provided in each of the operation unit 110L and the operation unit 110R. The servomotors SM7d, SM7e, and SM7f are examples of a first link motor, a second link motor, and a third link motor, respectively. The servomotor SM7g is an example of a grip support member motor.

The first control device 310 controls the servomotors via the operation controllers 340 to generate torques that counteract gravitational torques generated on rotation axes of the servomotors according to the postures of the operation units 110. Thus, the operator can operate the operation units 110 with a relatively small force.

When the operator performs an operation to rotate the grip support member 112d around the A27 axis of the operation unit 110 shown in FIGS. 12 and 13, the shaft 2d of the instrument 2 rotates around the A9 axis shown in FIG. 5. When the operator performs an operation to rotate the joints JT24, JT25, and JT26 of the operation unit 110 shown in FIGS. 12 and 13, the end effector 2b bends around the A10 axis or the A11 axis shown in FIG. 5. The shaft 2d has a rotational operating range around the A9 axis. The rotational operating range is defined, for example, as a rotatable angle from +A° to -A°. The rotational operating range of the shaft 2d includes a mechanical rotational operating range determined based on a machine constituting the shaft 2d and a rotational operating range defined on software preset in the robotic surgical system 500. In this embodiment, the rotational operating range of the shaft 2d defined on the software is smaller than the mechanical rotational operating range.

The operation controllers 340 are arranged in a main body of the remote control apparatus 200. The operation controllers 340 control the operation units 110. The operation controllers 340 are provided so as to correspond to the left-handed operation unit 110L and the right-handed operation unit 110R, respectively, as shown in FIG. 15.

As shown in FIG. 15, the vision unit 300 and the image processing unit 400 are connected to the first control device 310 through a LAN, for example. The display 220 is connected to the vision unit 300.

### Control for Operation out of Rotational Operating Range of Shaft

The control of the first control device 310 in a case in which an operation for the instrument 2 received by the operation unit 110 includes an operation to rotate the shaft 2d out of the rotational operating range of the shaft 2d is now described with reference to FIG. 19.

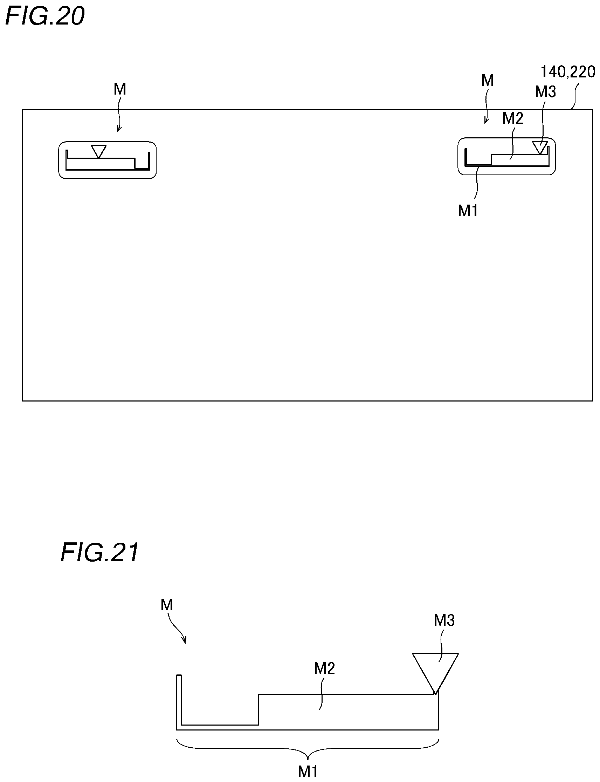

In step S1, the first control device 310 starts to receive an operation for the instrument 2 with the operation unit 110. In this embodiment, as shown in FIG. 20, the monitor 140 of the remote control apparatus 200 and the display 220 arranged on the cart 210 display an indicator M indicating the movable range M1 of the robot arm 50, the movable range M2 of the operation unit 110 with respect to the movable range M1 of the robot arm 50, and the current position M3 of the robot arm 50. The indicator M is displayed for each of the left-handed operation unit 110L and the right-handed operation unit 110R. For example, in an example shown in FIG. 21, the horizontal length of the movable range M2 of the operation unit 110 is smaller than the horizontal length of the movable range M1 of the robot arm 50, and the example indicates that the movable range M2 of the operation unit 110 is smaller than the movable range M1 of the robot arm 50. Furthermore, the current position M3 of the robot arm 50 has an inverted triangle shape, for example, and the position indicated by the inverted triangle shape represents the current position M3 of the robot arm 50.

In step S2, the operator performs an operation to rotate the grip support member 112d around the A27 axis of the operation unit 110 shown in FIG. 12 or 13, and the first control device 310 receives an operation to rotate the shaft 2d of the instrument 2 around the A9 axis shown in FIG. 5.

In step S3, in this embodiment, the first control device 310 determines whether or not the operation for the instrument 2 received by the operation unit 110 includes an operation to rotate the shaft 2d out of the rotational operating range of the shaft 2d. Specifically, the first control device 310 determines whether or not the operation for the instrument 2 received by the grip support member 112d of the operation unit 110 includes an operation to rotate the shaft 2d out of the rotational operating range of the shaft 2d. In this embodiment, a rotational operating range that serves as a criterion for the determination of the first control device 310 is a rotational operating range defined on the software that is smaller than the mechanical rotational operating range of the shaft 2d. Furthermore, the operation to rotate the shaft 2d received by the grip support member 112d of the operation unit 110 refers to an operation to rotate the grip support member 112d around the A27 axis. For example, when the rotatable angle is defined as +A° to -A°, and the operator attempts to rotate the grip support member 112d around the A27 axis to rotate the shaft 2d to an angle that exceeds an angular range from +A° to -A°, the first control device 310 determines yes in step S3.

In a case of yes in step S3, in this embodiment, in step S4, the first control device 310 restricts an operation to rotate the shaft 2d in a direction out of the rotational operating range with the operation unit 110 and an operation to bend the end effector 2b with respect to the shaft 2d with the operation unit 110. Specifically, when the operation for the instrument 2 includes an operation to rotate the shaft 2d out of the rotational operating range of the shaft 2d, the first control device 310 controls the servomotor SM7g to prevent rotation of the grip support member 112d. For example, when the rotatable angle of the shaft 2d is defined as +A° to -A°, and the operator attempts to rotate the grip support member 112d around the A27 axis to rotate the shaft 2d to an angle greater than +A°, the first control device 310 transmits a command to the servo controller SC7g to maintain the rotation angle of the shaft 2d at +A°. Thus, even when the operator attempts to rotate the grip support member 112d to rotate the shaft 2d in a direction greater than +A°, a relatively large load is applied to the grip support member 112d such that it is difficult for the grip support member 112d to rotate. In other words, when the operator attempts to rotate the grip support member 112d to rotate the shaft 2d in a direction greater than +A°, the servomotor SM7g generates a force to return the grip support member 112d to an angle of +A° such that it is difficult for the grip support member 112d to rotate. On the other hand, when the grip support member 112d is rotated to rotate the shaft 2d in a direction smaller than +A°, a load applied to the grip support member 112d is small such that the operator can easily rotate the grip support member 112d.

In this embodiment, in a case of yes in step S3, in step S4, an operation to bend the end effector 2b with respect to the shaft 2d with the link 112a, the link 112b, and the link 112c is restricted. The operation to bend the end effector 2b with respect to the shaft 2d includes an operation to rotate the second support member 2f around the A10 axis and an operation to rotate the end effector 2b around the A11 axis. Specifically, when the operation for the instrument 2 received by the operation unit 110 includes an operation to rotate the shaft 2d out of the rotational operating range of the shaft 2d, the first control device 310 restricts rotation of the link 112a around the A24 axis, rotation of the link 112b around the A25 axis, and rotation of the link 112c around the A26 axis. More specifically, when the operation for the instrument 2 received by the operation unit 110 includes an operation to rotate the shaft 2d out of the rotational operating range of the shaft 2d, the first control device 310 controls the servomotors SM7d, SM7e, and SM7f to prevent rotation of the links 112a, 112b, and 112c. For example, in a case of yes in step S3, the first control device 310 transmits commands to the servo controllers SC7d, SC7e, and SC7f to maintain the rotation angles of the links 112a, 112b, and 112c at the angles at the time at which the determination of "yes" is made in step 103. Thus, even when the operator attempts to rotate the links 112a, 112b, and 112c, a relatively large load is applied to the links 112a, 112b, and 112c such that it is difficult for the links 112a, 112b, and 112c to rotate. In other words, in a case of yes in step S3, the servomotors SM7d, SM7e, and SM7f generate forces to return the links 112a, 112b, and 112c to the angles at the time at which the determination of "yes" is made in step 103 such that it is difficult for the links 112a, 112b, and 112c to rotate.

Even in step S4 in which the operation for the instrument 2 received by the operation unit 110 includes an operation to rotate the shaft 2d out of the rotational operating range of the shaft 2d, an operation on the arm 111 of the operation unit 110 is continuously received. Furthermore, an operation to open and close the jaw members 2g and 2h of the instrument 2 by opening and closing the grip members 112e of the operation unit 110 is received.

As shown in FIG. 21, in a case of yes in step S3, the current position M3 of the robot arm 50 shown on the monitor 140 of the remote control apparatus 200 and the display 220 arranged on the cart 210 has moved to an end of the movable range M2 of the operation unit 110. By visually checking the monitor 140 or the display 220, the operator can visually recognize that the limit of the movable range M2 of the operation unit 110 has been reached.

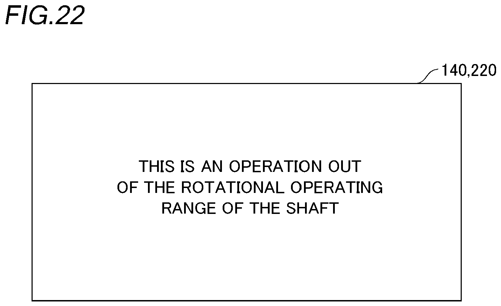

In this embodiment, in step S5, as shown in FIG. 22, when the operation for the instrument 2 received by the operation unit 110 includes an operation to rotate the shaft 2d out of the rotational operating range of the shaft 2d, the monitor 140 of the remote control apparatus 200 and the display 220 arranged on the cart 210 display a message indicating that the operation on the operation unit 110 is an operation to rotate the shaft 2d out of the rotational operating range of the shaft 2d. For example, the first control device 310 executes a process to display the message that the operation is an operation to rotate the shaft 2d out of the rotational operating range on the monitor 140 of the remote control apparatus 200 and the display 220 arranged on the cart 210.

In a case of no in step S3, the process returns to step S1. The operations from step S1 to step S5 are repeated while an operation with the operation unit 110 is being received.

### Advantages of This Embodiment

The first control device 310 is configured or programmed to restrict an operation to rotate the shaft 2d out of the rotational operating range of the shaft 2d with the operation unit 110 and an operation to bend the end effector 2b with respect to the shaft 2d with the operation unit 110 when the operation for the instrument 2 received by the operation unit 110 includes an operation to rotate the shaft 2d out of the rotational operating range of the shaft 2d. Accordingly, even when an operation is performed to rotate the shaft 2d out of the rotational operating range of the shaft 2d, an operation to rotate the shaft 2d out of the rotational operating range of the shaft 2d with the operation unit 110 and an operation to bend the end effector 2b with respect to the shaft 2d with the operation unit 110 are restricted, and thus the posture of the operation unit 110 is maintained. Therefore, the possibility that the postures of the end effector 2b and the shaft 2d do not match the posture of the operation unit 110 is reduced or prevented, and thus a state in which the postures of the end effector 2b and the shaft 2d match the posture of the operation unit 110 is maintained. In addition, the first control device 310 only restricts an operation to rotate the shaft 2d out of the rotational operating range with the operation unit 110 and an operation to bend the end effector 2b with respect to the shaft 2d with the operation unit 110, but does not restrict reception of all operations with the operation unit 110. Therefore, even when an operation is performed that attempts to exceed the movable range of the instrument 2, a sudden change in the posture of the instrument 2 can be reduced or prevented while a state in which an operation with the operation unit 110 can be received is maintained.

The rotational operating range of the shaft 2d is a rotational operating range defined on software that is smaller than the mechanical rotational operating range of the shaft 2d. Accordingly, the shaft 2d is prevented from operating beyond the mechanical rotational operating range, and thus mechanical failure of the instrument 2 can be prevented.

The instrument 2 includes the first support member 2e attached to the shaft 2d, and the second support member 2f supported by the first support member 2e so as to be rotatable around the A10 axis to support the end effector 2b such that the end effector 2b is rotatable around the A11 axis intersecting with the A10 axis as viewed in the direction in which the shaft 2d extends. The operation to bend the end effector 2b with respect to the shaft 2d includes an operation to rotate the second support member 2f around the A10 axis and an operation to rotate the end effector 2b around the A11 axis. Accordingly, when the operation for the instrument 2 received by the operation unit 110 includes an operation to rotate the shaft 2d out of the rotational operating range of the shaft 2d, rotation of the second support member 2f around the A10 axis and rotation of the end effector 2b around the A11 axis can be restricted.

The operation unit 110 includes the grip support member 112d to rotatably support one ends of the grip members 112e to be operated by the fingers of the operator and configured to rotate around the A27 axis parallel to the longitudinal direction of the grip support member 112d to receive an operation to rotate the shaft 2d. An operation to rotate the shaft 2d of the instrument 2 is received by rotating the grip support member 112d around the A27 axis. Accordingly, an operation to rotate the grip support member 112d out of the rotational operating range of the shaft 2d is restricted, and thus the possibility that the rotation angle of the grip support member 112d does not match the rotation angle of the shaft 2d can be reduced or prevented.

The operation unit 110 includes the servomotor SM7g to rotate the grip support member 112d around the A27 axis. The first control device 310 is configured or programmed to control the servomotor SM7g to prevent rotation of the grip support member 112d when the operation for the instrument 2 includes an operation to rotate the shaft 2d out of the rotational operating range of the shaft 2d. Accordingly, rotation of the grip support member 112d can be prevented by the prearranged servomotor SM7g without the need to separately arrange a dedicated brake or the like for preventing rotation of the grip support member 112d. Consequently, the complexity of the configuration of the robotic surgical system 500 can be reduced or prevented.

The operation unit 110 includes the arm 111 and the wrist 112. The wrist 112 includes the link 112a including the proximal end connected to the distal end of the arm 111 and rotatable around the A24 axis, the link 112b including the proximal end connected to the distal end of the link 112a and rotatable around the A25 axis, and the link 112c including the proximal end connected to the distal end of the link 112b and the distal end to which the grip support member 112d is connected and rotatable around the A26 axis. The first control device 310 is configured or programmed to restrict rotation of the link 112a around the A24 axis, rotation of the link 112b around the A25 axis, and rotation of the link 112c around the A26 axis when the operation for the instrument 2 includes an operation to rotate the shaft 2d out of the rotational operating range of the shaft 2d. Accordingly, rotation of the links 112a, 112b, and 112c is restricted, and thus the possibility that the postures of the links 112a, 112b, and 112c do not match the posture of the instrument 2 can be reduced or prevented.

The wrist 112 includes the servomotor SM7d to rotate the link 112a around the A24 axis, the servomotor SM7e to rotate the link 112b around the A25 axis, and the servomotor SM7f to rotate the link 112c around the A26 axis. The first control device 310 is configured or programmed to control the servomotors SM7d, SM7e, and SM7f to prevent rotation of the links 112a, 112b, and 112c when the operation for the instrument 2 includes an operation to rotate the shaft 2d out of the rotational operating range of the shaft 2d. Accordingly, rotation of the grip support member 112d can be prevented by the prearranged servomotors SM7d, SM7e, and SM7f without the need to separately arrange a dedicated brake or the like for preventing rotation of the links 112a, 112b, and 112c. Consequently, the complexity of the configuration of the robotic surgical system 500 can be reduced or prevented.

The monitor 140 of the remote control apparatus 200 and the display 220 arranged on the cart 210 are configured to display the message indicating that the operation on the operation unit 110 is an operation to rotate the shaft 2d out of the rotational operating range of the shaft 2d when the operation for the instrument 2 received by the operation unit 110 includes an operation to rotate the shaft 2d out of the rotational operating range of the shaft 2d. Accordingly, by visually checking the message, the operator can easily recognize that the operation on the operation unit 110 is an operation out of the rotational operating range of the shaft 2d.

The monitor 140 of the remote control apparatus 200 and the display 220 arranged on the cart 210 are configured to display the indicator M indicating the movable range M1 of the robot arm 50, the movable range M2 of the operation unit 110 with respect to the movable range M1 of the robot arm 50, and the current position M3 of the robot arm 50. Accordingly, by visually checking the current position M3 of the robot arm 50, the operator can easily recognize whether or not the operation on the operation unit 110 has reached the limit of the rotational operating range of the shaft 2d.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the remote control apparatus 200 includes two operation units 110 to operate two robot arms 50 in the aforementioned embodiment, the present disclosure is not limited to this. In the present disclosure, only one operation unit 110 may alternatively be provided in the remote control apparatus 200 to operate one robot arm 50.

While as the controller according to the present disclosure, the first control device 310 arranged in the medical cart 10 is applied in the aforementioned embodiment, the present disclosure is not limited to this. In the present disclosure, a controller other than the first control device 310 may alternatively be applied as the controller according to the present disclosure.

While the rotational operating range of the shaft 2d defined on the software is smaller than the mechanical rotational operating range in the aforementioned embodiment, the present disclosure is not limited to this. In the present disclosure, the rotational operating range of the shaft 2d defined on the software may alternatively be the same as the mechanical rotational operating range.

While the grip support member 112d of the operation unit 110 receives an operation to rotate the shaft 2d in the aforementioned embodiment, the present disclosure is not limited to this. In the present disclosure, an operation to rotate the shaft 2d may alternatively be received by a structure other than the grip support member 112d.

While the drive force of the servomotor SM7g acts to prevent rotation of the grip support member 112d in the aforementioned embodiment, the present disclosure is not limited to this. In the present disclosure, a brake may alternatively be provided on the servomotor SM7g to stop rotation of the servomotor SM7g.

While the first control device 310 performs a control to prevent rotation of the links 112a, 112b, and 112c of the wrist 112 when the operation for the instrument 2 received by the operation unit 110 includes an operation to rotate the shaft 2d out of the rotational operating range of the shaft 2d in the aforementioned embodiment, the present disclosure is not limited to this. For example, the first control device 310 may alternatively perform a control to increase resistance to an operation to rotate the links 112a, 112b, and 112c of the wrist 112. That is, while the links 112a, 112b, and 112c are capable of rotating, resistance to an operation outside the rotational operating range of the shaft 2d is increased as compared with resistance to an operation within the rotational operating range of the shaft 2d. Alternatively, it may be possible to select, for each axis of the wrist 112, a control to prevent the rotation or a control to increase the resistance.

While an operation on the arm 111 of the operation unit 110 is continuously received even when the operation for the instrument 2 received by the operation unit 110 includes an operation to rotate the shaft 2d out of the rotational operating range of the shaft 2d in the aforementioned embodiment, the present disclosure is not limited to this. In the present disclosure, when the operation for the instrument 2 received by the operation unit 110 includes an operation to rotate the shaft 2d out of the rotational operating range of the shaft 2d, a control may alternatively be performed to prevent rotation of the links 111a, 111b, and 111c of the arm 111.

While the operation unit 110 includes seven axes of A21 to A27 in the aforementioned embodiment, the present disclosure is not limited to this. In the present disclosure, the number of axes of the operation unit 110 may alternatively be other than seven. Furthermore, while the wrist 112 of the operation unit 110 includes four axes of A24 to A27 in the aforementioned embodiment, the present disclosure is not limited to this. In the present disclosure, the number of axes of the wrist 112 may alternatively be other than four, such as three or five.

While the wrist 112 of the operation unit 110 has a gimbal structure in which the A24 and A26 axes, the A25 axis, and the A27 axis are mutually perpendicular in the aforementioned embodiment, the present disclosure is not limited to this. In the present disclosure, the wrist 112 may not have a gimbal structure. That is, the axes of the wrist 112 may not be mutually perpendicular.

While operations with the three links 112a, 112b, and 112c are restricted when the operation for the instrument 2 received by the operation unit 110 includes an operation to rotate the shaft 2d out of the rotational operating range of the shaft 2d in the aforementioned embodiment, the present disclosure is not limited to this. In the present disclosure, operations with a number of links other than three may alternatively be restricted.

While the drive forces of the servomotors SM7d, SM7e, and SM7f act to prevent rotation of the links 112a, 112b, and 112c in the aforementioned embodiment, the present disclosure is not limited to this. In the present disclosure, a brake may alternatively be provided on each of the links 112a, 112b, and 112c to stop rotation of links 112a, 112b, and 112c.

While the message indicating that the operation on the operation unit 110 is an operation out of the rotational operating range of the shaft 2d is displayed on the monitor 140 of the remote control apparatus 200 and the display 220 arranged on the cart 210 in the aforementioned embodiment, the present disclosure is not limited to this. In the present disclosure, the above message may alternatively be displayed on only one of the monitor 140 and the display 220.

While the indicator M indicating the movable range M1 of the robot arm 50, the movable range M2 of the operation unit 110, and the current position M3 of the robot arm 50 is displayed on the monitor 140 of the remote control apparatus 200 and the display 220 arranged on the cart 210 in the aforementioned embodiment, the present disclosure is not limited to this. In the present disclosure, the above indicator M may alternatively be displayed on only one of the monitor 140 and the display 220.

While four robot arms 50 are provided in the aforementioned embodiment, the present disclosure is not limited to this. In the present disclosure, the number of robot arms 50 may alternatively be any number as long as there is at least one.

While each of the arm portion 51 and the positioner 30 includes a 7-axis articulated robot in the aforementioned embodiment, the present disclosure is not limited to this. For example, each of the arm portion 51 and the positioner 30 may alternatively include an articulated robot having an axis configuration other than the 7-axis articulated robot. The axis configuration other than the 7-axis articulated robot refers to six axes or eight axes, for example.

While the surgical robot 100 includes the medical cart 10, the positioner 30, the arm base 40, and the robot arms 50 in the aforementioned embodiment, the present disclosure is not limited to this. For example, the surgical robot 100 may not include the medical cart 10, the positioner 30, or the arm base 40, but may include only the robot arms 50.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

### Aspects

It will be appreciated by those skilled in the art that the exemplary embodiments described above are specific examples of the following aspects.

### (Item 1)

A robotic surgical system comprising:
a robot arm to allow a surgical instrument including a shaft and a distal end device connected to a distal end of the shaft via a wrist joint to be attached thereto;
an operation apparatus including an operation unit to receive an operation for the surgical instrument; and
a controller configured or programmed to restrict an operation to rotate the shaft in a direction out of a rotational operating range of the shaft with the operation unit and an operation to bend the distal end device with respect to the shaft with the operation unit when the operation for the surgical instrument received by the operation unit includes an operation to rotate the shaft out of the rotational operating range of the shaft.

### (Item 2)

The robotic surgical system according to item 1, wherein the rotational operating range of the shaft is a rotational operating range defined on software that is smaller than a mechanical rotational operating range of the shaft.

### (Item 3)

The robotic surgical system according to item 1 or 2, wherein
the surgical instrument includes a first support member attached to the shaft, and a second support member supported by the first support member so as to be rotatable around a first rotation axis to support the distal end device such that the distal end device is rotatable around a second rotation axis intersecting with the first rotation axis as viewed in a direction in which the shaft extends; and
the operation to bend the distal end device with respect to the shaft includes an operation to rotate the second support member around the first rotation axis and an operation to rotate the distal end device around the second rotation axis.

### (Item 4)

The robotic surgical system according to any one of items 1 to 3, wherein
the operation unit includes a grip support member to rotatably support one end of a grip member to be operated by a finger of an operator, the grip support member being configured to rotate around a third rotation axis parallel to a longitudinal direction of the grip support member to receive an operation to rotate the shaft; and
the operation to rotate the shaft is received by rotating the grip support member around the third rotation axis.

### (Item 5)

The robotic surgical system according to item 4, wherein
the operation unit includes a grip support member motor to rotate the grip support member around the third rotation axis; and
the controller is configured or programmed to control the grip support member motor to prevent rotation of the grip support member when the operation for the surgical instrument includes the operation to rotate the shaft out of the rotational operating range of the shaft.

### (Item 6)

The robotic surgical system according to item 4 or 5, wherein
the operation unit includes an arm and a wrist;
the wrist includes:
   a first link including a proximal end connected to a distal end of the arm and rotatable around a fourth rotation axis;
   a second link including a proximal end connected to a distal end of the first link and rotatable around a fifth rotation axis; and
   a third link including a proximal end connected to a distal end of the second link and a distal end to which the grip support member is connected and rotatable around a sixth rotation axis; and
the controller is configured or programmed to restrict rotation of the first link around the fourth rotation axis, rotation of the second link around the fifth rotation axis, and rotation of the third link around the sixth rotation axis when the operation for the surgical instrument received by the operation unit includes the operation to rotate the shaft out of the rotational operating range of the shaft.

### (Item 7)

The robotic surgical system according to item 6, wherein
the wrist includes:
   a first link motor to rotate the first link around the fourth rotation axis;
   a second link motor to rotate the second link around the fifth rotation axis; and
   a third link motor to rotate the third link around the sixth rotation axis; and
the controller is configured or programmed to control the first link motor, the second link motor, and the third link motor to prevent rotation of the first link, the second link, and the third link when the operation for the surgical instrument received by the operation unit includes the operation to rotate the shaft out of the rotational operating range of the shaft.

### (Item 8)

The robotic surgical system according to any one of items 1 to 7, comprising:
a first display to display a message indicating that an operation on the operation unit is the operation to rotate the shaft out of the rotational operating range of the shaft when the operation for the surgical instrument received by the operation unit includes the operation to rotate the shaft out of the rotational operating range of the shaft.

### (Item 9)

The robotic surgical system according to any one of items 1 to 8, comprising:
a second display to display an indicator indicating a movable range of the robot arm, a movable range of the operation unit with respect to the movable range of the robot arm, and a current position of the robot arm.

### (Item 10)

A control method for a robotic surgical system, the robotic surgical system comprising a robot arm to allow a surgical instrument including a shaft and a distal end device connected to a distal end of the shaft via a wrist joint to be attached thereto, and an operation apparatus including an operation unit to receive an operation for the surgical instrument, the control method comprising:
receiving the operation for the surgical instrument by the operation unit; and
restricting an operation to rotate the shaft in a direction out of a rotational operating range of the shaft with the operation unit and an operation to bend the distal end device with respect to the shaft with the operation unit when the operation for the surgical instrument received by the operation unit includes an operation to rotate the shaft out of the rotational operating range of the shaft.

### (Item 11)

A program for the control method for the robotic surgical system according to item 10.

### (Item 12)

A storage medium configured to store the program for the control method for the robotic surgical system according to item 11.

## Claims

1. A robotic surgical system (500) comprising:
a robot arm (50) to allow a surgical instrument (2) including a shaft (2d) and a distal end device (2b) connected to a distal end of the shaft via a wrist joint (2c) to be attached thereto;
an operation apparatus (200) including an operation unit (110) to receive an operation for the surgical instrument; and
a controller (310) configured or programmed to restrict an operation to rotate the shaft in a direction out of a rotational operating range of the shaft with the operation unit and an operation to bend the distal end device with respect to the shaft with the operation unit when the operation for the surgical instrument received by the operation unit includes an operation to rotate the shaft out of the rotational operating range of the shaft.

2. The robotic surgical system according to claim 1, wherein the rotational operating range of the shaft is a rotational operating range defined on software that is smaller than a mechanical rotational operating range of the shaft.

3. The robotic surgical system according to claim 1 or 2, wherein
the surgical instrument includes a first support member (2e) attached to the shaft, and a second support member (2f) supported by the first support member so as to be rotatable around a first rotation axis (A10) to support the distal end device such that the distal end device is rotatable around a second rotation axis (A11) intersecting with the first rotation axis as viewed in a direction in which the shaft extends; and
the operation to bend the distal end device with respect to the shaft includes an operation to rotate the second support member around the first rotation axis and an operation to rotate the distal end device around the second rotation axis.

4. The robotic surgical system according to any one of claims 1 to 3, wherein
the operation unit includes a grip support member (112d) to rotatably support one end of a grip member (112e) to be operated by a finger of an operator, the grip support member being configured to rotate around a third rotation axis (A27) parallel to a longitudinal direction of the grip support member to receive an operation to rotate the shaft; and
the operation to rotate the shaft is received by rotating the grip support member around the third rotation axis.

5. The robotic surgical system according to claim 4, wherein
the operation unit includes a grip support member motor (SM7g) to rotate the grip support member around the third rotation axis; and
the controller is configured or programmed to control the grip support member motor to prevent rotation of the grip support member when the operation for the surgical instrument includes the operation to rotate the shaft out of the rotational operating range of the shaft.

6. The robotic surgical system according to claim 4 or 5, wherein
the operation unit includes an arm (111) and a wrist (112) ;
the wrist includes:
a first link (112a) including a proximal end connected to a distal end of the arm and rotatable around a fourth rotation axis (A24);
a second link (112b) including a proximal end connected to a distal end of the first link and rotatable around a fifth rotation axis (A25); and
a third link (112c) including a proximal end connected to a distal end of the second link and a distal end to which the grip support member is connected and rotatable around a sixth rotation axis (A26); and
the controller is configured or programmed to restrict rotation of the first link around the fourth rotation axis, rotation of the second link around the fifth rotation axis, and rotation of the third link around the sixth rotation axis when the operation for the surgical instrument received by the operation unit includes the operation to rotate the shaft out of the rotational operating range of the shaft.

7. The robotic surgical system according to claim 6, wherein
the wrist includes:
a first link motor (SM7d) to rotate the first link around the fourth rotation axis;
a second link motor (SM7e) to rotate the second link around the fifth rotation axis; and
a third link motor (SM7f) to rotate the third link around the sixth rotation axis; and
the controller is configured or programmed to control the first link motor, the second link motor, and the third link motor to prevent rotation of the first link, the second link, and the third link when the operation for the surgical instrument received by the operation unit includes the operation to rotate the shaft out of the rotational operating range of the shaft.

8. The robotic surgical system according to any one of claims 1 to 7, comprising:
a first display (140, 220) to display a message indicating that an operation on the operation unit is the operation to rotate the shaft out of the rotational operating range of the shaft when the operation for the surgical instrument received by the operation unit includes the operation to rotate the shaft out of the rotational operating range of the shaft.

9. The robotic surgical system according to any one of claims 1 to 8, comprising:
a second display to display an indicator indicating a movable range of the robot arm, a movable range of the operation unit with respect to the movable range of the robot arm, and a current position of the robot arm.

10. A control method for a robotic surgical system (500), the robotic surgical system comprising a robot arm (50) to allow a surgical instrument (2) including a shaft (2d) and a distal end device (2b) connected to a distal end of the shaft via a wrist joint (2c) to be attached thereto, and an operation apparatus (200) including an operation unit (110) to receive an operation for the surgical instrument, the control method comprising:
receiving the operation for the surgical instrument by the operation unit; and
restricting an operation to rotate the shaft in a direction out of a rotational operating range of the shaft with the operation unit and an operation to bend the distal end device with respect to the shaft with the operation unit when the operation for the surgical instrument received by the operation unit includes an operation to rotate the shaft out of the rotational operating range of the shaft.

11. A program for the control method for the robotic surgical system (500) according to claim 10.

12. A storage medium configured to store the program for the control method for the robotic surgical system (500) according to claim 11.
